# EUROPEAN PATENT SPECIFICATION

(11) **EP 0 595 928 B1**
(45) Date of publication and mention of the grant of the patent: **09.04.1997**
(21) Application number: 92915932.5
(22) Date of filing: 20.07.1992
(51) Int. Cl.: A61F 13/15, A61L 15/16, B65D 65/38

(54) **FLEXIBLE ABSORBENT SHEET**
BIEGSAME ABSORBIERENDE SCHICHT
FEUILLE ABSORBANTE SOUPLE

(30) Priority: 19.07.1991 US 732852
(43) Date of publication of application: 11.05.1994
(73) Proprietor: JOHNSON & JOHNSON INC., Montreal, Quebec H1N 2G4 (CA)
(72) Inventor: CHAUVETTE, Gaétan, St-Hubert, Quebec J3Y 8Z3 (CA); RAMACIERI, Patricia, Montreal, Quebec H1E 3J4 (CA)
(74) Representative: Strehl Schübel-Hopf Groening & Partner
(86) International application number: PCT/CA92/00307
(87) International publication number: WO 93/01730

(56) References cited:
- EP-A- 0 342 646
- EP-A- 0 458 657
- US-A- 4 144 122
- US-A- 4 559 050
- US-A- 4 596 567
- US-A- 4 853 086

## Description

### FIELD OF THE INVENTION

The invention relates to the art of manufacturing structures for absorbing body exudate. More specifically, the invention relates to a highly absorbent, flexible and resilient, non-defibrated cellulosic board providing a soft, fluid-absorbent component, well-suited for use in disposable absorbent products such as sanitary napkins, tampons, diapers, incontinence pads, adult briefs, wound dressings and the like. The invention also extends to a method for manufacturing the non-defibrated cellulosic board.

### BACKGROUND OF THE INVENTION

Many disposable absorbent articles use absorbent cores made primarily of cellulosic pulp fluff material. Such cores are generally soft, flexible and absorbent but tend to be bulky and thick and have poor wicking properties. In addition, cellulosic pulp fluff material has poor structural stability which may cause the absorbent core to collapse when saturated with fluid.

An absorbent structure that has poor wicking properties may increase the likelihood of failure of the absorbent product to hold and contain body fluids. Body exudate will be localized to a certain area of a poorly wicking absorbent core, causing saturation in such area whereby excess fluid may overflow through an external surface of the absorbent product. This overflow may contact the user's garment and cause stains or contact the user's body and cause wet discomfort or rash. It is therefore desirable to provide an absorbent core for disposable absorbent articles which can wick away body fluids from the point of contact with the absorbent core and spread it through the absorbent core to more efficiently utilize the entire surface area of the absorbent core. The improved wicking properties of such an absorbent core provide the capacity for fluids to travel by capillary pressure throughout the surface area of the absorbent core and thus permit the use of thinner cores, since more absorbent volume can be made available for absorbing body fluids by such wicking action. Thinner absorbent cores may prove to be more comfortable for the user and less unsightly or obvious when worn under clothes.

Absorbent cores with excellent wicking properties comprising peat moss and wood pulp composite materials are described, for example, in the following U.S. patents:

| **PATENT #** | **INVENTOR(s)** | **DATE OF ISSUE** |
|---|---|---|
| 4,170,515 | Lalancette et al. | October 9, 1979 |
| 4,215,692 | Levesque | August 5, 1980 |
| 4,226,237 | Levesque | October 7, 1980 |
| 4,305,393 | Nguyen | December 15, 1981 |
| 4,473,440 | Ovans | September 25, 1984 |
| 4,507,122 | Levesque | March 26, 1985 |
| 4,618,496 | Brasseur | October 21, 1986 |
| 4,676,871 | Cadieux et al. | June 30, 1987 |
| 4,992,324 | Dubé | February 12, 1991 |
| 5,053,029 | Yang | October 1, 1991 |

The subject matter of these patents is incorporated herein by reference.

In accordance with the teaching of these patents, an absorbent structure comprising peat moss as a primary absorbent component is formed as a sheet by air or wet laying of fibers. The sheet is calendered to obtain a relatively thin, i.e. from about 0.025 to 0.25 centimeters (cm) thick and relatively dense, i.e. from about 0.2 to 1.0 grams per cubic centimeter (g/cc) structure. Such absorbent peat moss sheet may be processed to increase its flexibility for enhancing its comfort potential by subjecting the sheet to mechanical tenderizing such as by a perf-embossing process.

The peat moss sheet thus formed has a large proportion of extremely tiny pores and capillaries which allow the sheet to absorb and retain an enormous capacity of fluid. The peat moss pores swell as they absorb fluid, however, this swelling does not cause a loss of capacity for further absorbing fluid. Rather, the swelling contributes to the ability of the sheet to retain fluid while generally maintaining the structural integrity of the absorbent structure in use.

The wicking properties of the above-described peat moss sheet provide the ability for the sheet to be highly absorbent while remaining relatively thin.

While peat moss sheets make excellent absorbent and wicking cores for disposable absorbent products, they cannot be economically produced in areas which lack the critical raw material, i.e. peat moss or sphagnum moss of desirable age, structure and moisture content. Therefore, there exists a need to provide a thin, highly absorbent and wicking material suitable for manufacturing absorbent cores for disposable absorbent articles, which forms an acceptable substitute to peat moss material.

Attempts to utilize other cellulosic materials such as Kraft wood pulp boards as absorbent cores have not been successful because they tend not to have as much absorbent capacity as peat moss composite sheets but more importantly, Kraft wood pulp boards cannot be sufficiently softened for their intended use. While the flexibility and other characteristics of such Kraft wood pulp boards may be improved by perf-embossing, such products still do not provide a desirable combination of absorption capacity, fluid penetration rate, wicking rate and most importantly a sufficient degree of flexibility for optimal use in disposable absorbent products.

### OBJECTS OF THE INVENTION

It is, therefore, an object of the present invention to provide a fluid-absorbent cellulosic sheet which does not utilize peat moss as a primary absorbent medium, yet it has a sufficient absorption capacity as well as a relatively short fluid acceptance time, and possesses good flexibility and resiliency for use in disposable absorbent articles, particularly for sanitary usage. Optimal flexibility of such products requires that the product be comfortably soft and flexible to the wearer but stiff and strong enough to withstand bunching and breakage when subjected to mechanical stress in a dry and in a wet state.

Another object of the invention is to provide a method for manufacturing the aforementioned fluid-absorbent cellulosic sheet.

### SUMMARY OF THE INVENTION

Traditionally, cellulosic pulp fluff has been manufactured by subjecting a dense cellulosic pulp board to intense mechanical working for rupturing the physical interfiber bonds to form a fibrous network having a high void volume, the so-called "fluff". This operation is commonly referred to as "defibration" and it is performed with a grinding mill or with any other suitable defibrator. An important economic factor in any process to convert cellulosic pulp boards into fluff material is the energy cost for operating the defibrator. It is generally accepted that regardless of the kind of equipment used for the conversion, the cost of energy is a significant factor in the overall conversion expenditure.

To reduce the energy consumption of a defibrator, it is common practise to incorporate in the cellulosic pulp board a debonding agent which acts to reduce the forces uniting the cellulosic fibers. As a result, less energy is required to defibrate the cellulosic pulp board. The most popular and widely used debonding agents which are commercially available from various sources are based on quaternary ammonium compounds. Canadian patent 1,152,710 granted to Kimberly-Clark Corporation on August 30, 1983, describes a debonder of this class. Debonding agents are also described and disclosed in U.S. patent 4,482,429, U.S. patent 3,972,855, U.S. patent 4,144,122 and U.S. patent 4,432,833.

It has also been suggested in the past to subject a cellulosic pulp board including debonding to perf-emboss to reduce the stiffness of the board to acceptable levels for use as an absorbent core in a disposable absorbent product. The combination of debonding agent and perf-embossing increases the absorption and flexibility of the cellulosic pulp board.

As a substitute to chemical debonding agents, it has been suggested to introduce in the cellulosic pulp board cross-linked cellulosic fibers providing a high-bulk, resilient structure acting to maintain a certain spacing between the fibers of the cellulosic material, to achieve a reduction in the cohesiveness of the fibrous network. As an example, the United States patent number 4,853,086 granted to Weyerhaueser Company on August 1, 1989 describes a method for manufacturing such cross-linked cellulosic fibers. In essence, the method consists of spraying a wet or partially dried web of cellulosic fibers with an aqueous solution of a glycol and dialdehyde.

The present inventors have made the unexpected discovery that by incorporating a debonding agent (for the purpose of this specification "debonding agent" should be construed to include any agency acting chemically on the cellulose fibers to reduce the incidence of hydrogen bonding between the fibers) and cross-linked cellulosic fibers into a board of cellulosic fibrous material, a synergy effect develops, vastly improving the resiliency, flexibility and fluid-absorbency (for the purpose of this specification, "fluid-absorbency" shall solely mean the ability of a body to take-up fluid, regardless of how fluid-retentive the body is. For example, the transfer layer of a compound absorbent structure provided to meter fluid to a reservoir layer, will be described as fluid-absorbent although it has a relatively poor fluid retentivity) of the board, providing a soft, resilient and highly absorbent sheet that may be used in a non-defibrated form (for the purpose of this specification, "non-defibrated" shall mean the absence of a fluffed condition. More specifically, this expression would describe a fibrous material formed as a dense, non-fluffed product which has not been subjected to any mechanical treatment for the purpose of separating the material in its fibrous constituents. This expression would also describe fluffed fibrous material which has been converted to a non-fluffed form) as an absorbent component for disposable absorbent products such as sanitary napkins, diapers, adult briefs, incontinence pads, wound dressing and the like. A particularly significant advantage of this absorbent structure resides in that no defibration is required, which considerably reduces the manufacturing cost of the absorbent structure.

The debonding agent cooperates with the cross-linked cellulosic fibers to relax the fibrous network of the cellulosic material. A possible explanation of this synergistic action, which is provided herein without any intent of limiting the scope of the invention to a specific theory, is that the debonding agent acts to weaken the interfiber bonds of the cellulosic fibrous material, while the cross-linked cellulosic fibers provide, by virtue of their inherent resiliency, an expansion force pushing the fibers away from one another. The resulting fibrous network has a void volume which is high enough to provide an excellent fluid-absorption capacity and a high fluid-acceptance rate, while remaining below the level beyond which the structural integrity of the fluid-absorbent sheet is compromised in use. The increase in the interfiber distances also provides the fibrous network with the ability to flex under low effort and to return to its original configuration upon cessation of the deformation effort. These characteristics provide increased comfort potential allowing the non-defibrated cellulosic board to be used as an absorbent component in disposable absorbent products for use next to the body.

In a preferred embodiment, the non-defibrated cellulosic board comprises cross-linked, fibrous cellulosic material in the range from about 20 to about 80 percent based on the weight of de-moisturized cellulose in the board. The debonding agent is present in the non-defibrated cellulosic board in the range from about 0.05 to about 5 percent based on the weight of the de-moisturized board.

Preferably, the non-defibrated cellulosic board is mechanically tenderized by perf-embossing in order to enhance its comfort potential.

The starting material for manufacturing the non-defibrated cellulosic board is preferably selected from the group consisting of sulfate, sulfite, debonded, bleached or unbleached wood pulp, thermo-mechanical pulp, chemical thermal mechanical pulp, ground wood, cotton linters and mixtures thereof.

As embodied and broadly described herein, the invention further provides a method for manufacturing a resilient and flexible, fluid-absorbent sheet, comprising the step of incorporating into a non-defibrated board of cellulosic fibrous material effective amounts of cross-linked cellulosic fibers and debonding agent.

In a preferred embodiment, the cross-linked cellulosic fibers are added to an aqueous slurry of cellulosic pulp which has been pre-treated with debonding agent. The slurry is then formed into a web and dewatered to obtain the non-defibrated cellulosic board.

### BRIEF DESCRIPTION OF THE DRAWINGS

- Figure 1 is an idealized perspective view at the fiber level of a non-defibrated cellulosic pulp board in raw form, i.e. free of agents that hinder the hydrogen bonding between the cellulosic fibers;
- Figure 2 illustrates the network of the cellulosic pulp board of Figure 1 in which cross-linked cellulosic fibers have been incorporated;
- Figure 3 is an idealized view of the fibrous network shown in Figure 2 in which a debonding agent has been incorporated;
- Figure 4 is a graphic illustration of the perf-embossing operation for tenderizing the non-defibrated cellulosic board in accordance with the invention;
- Figure 5 is a vertical cross-sectional view of the perforation rolls which constitute the first stage of the perf-embossing treatment;
- Figure 6 is a fragmentary front elevational view of the perforation rolls shown in Figure 6, the non-defibrated cellulosic board to be treated being omitted for illustrating the interrelation between the perforation teeth of the rolls;
- Figure 7 is a vertical cross-sectional view of the cross-direction embossing rolls which constitute the second stage of the perf-embossing treatment;
- Figure 8 is a top view of one of the cross-direction embossing roll, also showing the resulting embossing pattern created on the non-defibrated cellulosic board;
- Figure 9 is a vertical cross-sectional view of the machine-direction embossing rolls which constitute the third and last stage of the perf-embossing treatment;
- Figure 10 is a top view of one of the machine-direction embossing rolls, also showing the resulting embossing pattern created on the non-defibrated cellulosic board;
- Figure 11 is a schematical representation of a gravimetric absorbency test system (GATS);
- Figure 12 is a perspective view of a set-up for conducting a drying power test procedure;
- Figure 13 is a perspective view of a set-up for conducting a 45° impact capacity test procedure; and
- Figure 14 is a fragmentary, perspective view of a sanitary napkin incorporating the non-defibrated cellulosic board according to the invention.

### DESCRIPTION OF A PREFERRED EMBODIMENT

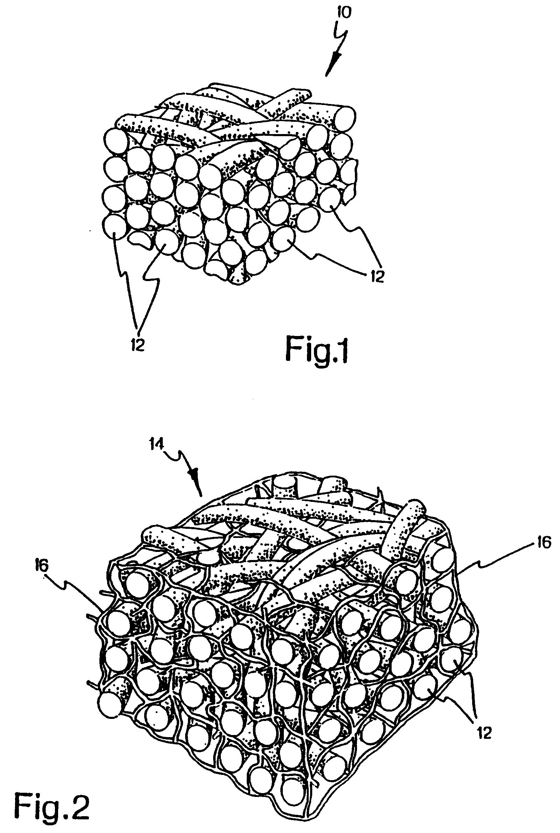

Figure 1 is an idealized, perspective view on a highly enlarged scale of the fibrous network 10 of a non-defibrated cellulosic pulp board in raw form. It should be understood that this representation is solely for illustrative purposes and it does not necessarily conform to the true material structure of the cellulosic pulp board.

The fibrous network 10 is composed of individual cellulosic fibers 12 which are randomly oriented and are united to one another by hydrogen bonding. The fibers 12 form a highly dense and cohesive network with only a limited amount of void volume therebetween. This translates into poor fluid-absorption properties and into a rigid, relatively non-conformable structure, making the cellulosic pulp board unsuitable for use as an absorbent layer in a disposable absorbent product, particularly for sanitary usage.

In order to reduce the cohesiveness of the fibrous network 10, primarily for facilitating its mechanical defibration, the prior art teaches to incorporate in the fibrous network 10 cross-linked cellulosic fibers providing a high-bulk, resilient structure which functions to physically space the fibers 12 from one another.

Figure 2 illustrates schematically the fibrous network 14 of a non-defibrated cellulosic pulp board containing cross-linked cellulosic fibers 16. The cross-linked fibers 16, forming a space frame-like structure are uniformly interspersed with the fibers 12 and, by virtue of their inherent resiliency, push to some degree the fibers 12 away from one another. However, it is believed that the hydrogen bonding between the fibers 12 remains very strong and greatly overwhelms the resistance to deformation of the cross-linked cellulosic fibers 16. As a result, the space frame-like structure is in a virtually collapsed condition, achieving only a limited reduction in cohesiveness of the fibrous network 14. The reduction in structural integrity of the fibrous network 14 considerably reduces the energy required to mechanically defibrate it, however, it does not sufficiently improve its fluid-absorbency, resiliency and flexibility to allow the fibrous network 14 to be advantageously used as an absorbent component in a disposable absorbent product for sanitary usage.

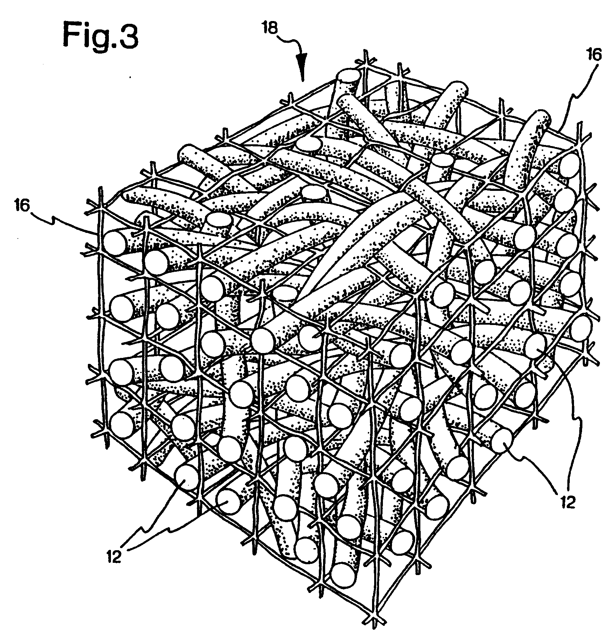

Figure 3 illustrates a non-defibrated cellulosic pulp board treated with a debonding agent and containing cross-linked cellulosic fibers 16. It is apparent that the fibrous network 18 is considerably more relaxed than the fibrous network 14, exhibiting comparatively large interfiber distances which translate into a higher void volume and in an increased overall bulk. The significant improvement in bulking by comparison to the fibrous network 14 shown in Figure 2 is believed to be donnated by the debonding agent which inhibits the hydrogen bonding between the fibers, reducing the forces normally collapsing the space frame-like structure formed by the cross-linked cellulosic fibers 16. As a result, the space framelike structure expands in all directions the entire fibrous network.

The fibrous network 18 is highly advantageous for use in a non-defibrated form as an absorbent component for a disposable absorbent product such as a sanitary napkin, a diaper, an incontinence pad, an adult brief, a wound dressing and the like. It offers exceptional fluid absorption properties such as a good capacity and a high fluid-acceptance rate as well as good wicking characteristics. In addition, it has an excellent comfort potential because it is soft and flexible, providing an absorbent product which is highly conformable to the area of the body to which it is intended to be applied, thus providing good gasketing properties, i.e. the ability to conform to the surface of the body and form a leak-proof seal. In addition, the fibrous network 18 is capable to maintain its structural integrity in the dry and in the wet states. Further, its degree of collapse upon taking-up fluid is relatively small, thereby providing a good structural integrity when soaked with fluid.

Some fluid-absorbency characteristics of the fibrous network 18 may be tailored according to the intended application by varying the density of the fibrous network 18. For example, by decreasing the density of the fibrous network 18, the fluid-acceptance rate increases at the expense of a reduction of the fluid retentivity. Such a structure would be suitable as a transfer layer in a compound absorbent component for metering fluid to a reservoir layer. Conversely, an increased density will favour the fluid-absorbency requirements for a reservoir layer. For a single layer absorbent structure, the density is selected to provide the necessary balance between the various fluid-absorbency characteristics.

A non-defibrated cellulosic board according to the invention is manufactured by incorporating into an aqueous slurry of cellulosic wood pulp material pretreated with debonding agent the cross-linked cellulosic fibers. The percentage of cross-linked cellulosic fibers in the slurry is in the range from about 20 to about 80 percent based on the weight of demoisturized cellulose in the slurry.

The method for manufacturing the cross-linked cellulosic fibers 16 will not be described herein because this technology is well documented in the patent literature. For example, U.S. patent 4,853,086 discloses a process for manufacturing the cross-linked cellulosic fibers by spraying a wet or a partially dried cellulosic fibrous web with an aqueous solution of a glycol and dialdehyde. The entire disclosure of this patent is incorporated herein by reference.

Cellulosic wood pulp material treated with debonding agent is a commercially available product, which can be obtained for example from the Weyerhaeuser Company under the name NBFA. This product is essentially Kraft wood pulp fibers incorporating a BEROCELL 584 brand debonding agent (commercially available from the Berol Chemie Company) in the range from about 0.3 to about 0.45 percent by weight of the de-moisturized product, and formed into a board by wet laying.

It is also possible to use untreated cellulosic wood pulp material, either in a defibrated or in a non-defibrated condition which is treated with debonding agent on site before the preparation of the aqueous slurry. The amount of debonding agent added to the cellulosic wood pulp material is selected so that in the non-defibrated cellulosic board (including the cross-linked cellulosic fibers) the debonding agent is present in the range from about 0.05 to about 5 percent by weight of the de-moisturized board. A hydrophilic debonder, has been found satisfactory. (By "hydrophilic debonder" is meant a debonding agent that preserves the hydrophilic nature of the cellulosic material).

The cellulosic wood pulp material treated with the selected debonding agent is converted to slurry by simple dispersion in water. The desired amount of cross-linked cellulosic fibers is added to the slurry and the process is completed by sheeting the aqueous slurry to form a web and by dewatering the web to form the non-defibrated cellulosic board. The last two operations are performed in accordance with conventional techniques for manufacturing wet-laid cellulosic boards, therefore a detailed description of these steps is not deemed necessary.

The dewatered web is subjected to a calendering operation in order to control the density of the final product. The calendering operation increases the density of the non-defibrated cellulosic board to enhance its fluid-retentivity and wicking power. As previously mentioned, by varying the density of the non-defibrated cellulosic board its fluid-absorbency characteristics can be tailored to suit specific applications.

The starting material which is to be treated with debonding agent for making the slurry of cellulosic material is selected from the group consisting of sulfate, sulfite, debonded, bleached or unbleached wood pulp, thermo-mechanical pulp, chemical thermal mechanical pulp, ground wood, cotton linters and mixtures thereof.

In order to further tenderize, soften and improve the flexibility of the non-defibrated cellulosic board, it is subjected to mechanical tenderizing by perf-embossing.

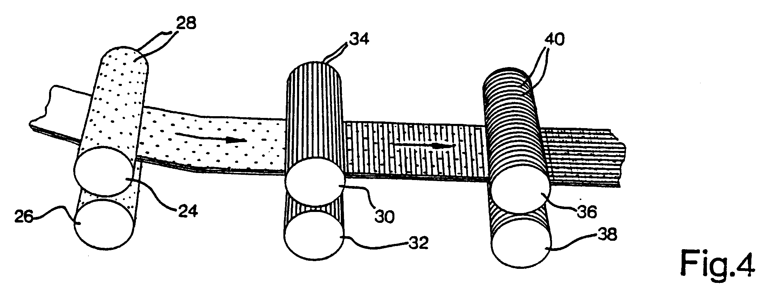

The mechanical tenderizing operation is graphically depicted in Figure 4. Generally stated, the perf-embossing technique first perforates the non-defibrated cellulosic board, then sequentially embosses the resulting material in the Y (cross-direction) and X (machine direction).

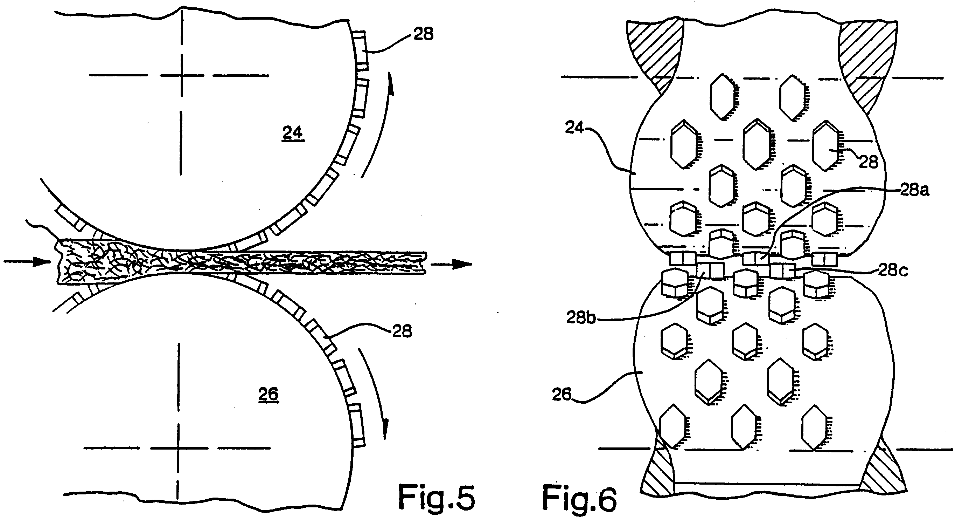

The "PERF" operation (first step), best illustrated in Figures 4, 5 and 6, is performed by passing the non-defibrated cellulosic board between a pair of rolls 24 and 26 provided with intermeshing and non-contacting teeth 28 perforating the material by shearing action to open-up its structure. The teeth 28 on the companion rolls 24,26 are so arranged that tooth 28a on top roll 24 is off-center the inter-teeth void defined between adjacent and axially aligned teeth 28b and 28c. The shearing action actually occurs between teeth 28a,28c during intermeshing, locally perforating the non-defibrated cellulosic board.

In a preferred embodiment, the interference i.e. the overlap between the teeth 28 of the perforating rolls 24 and 26 is set at approximately 1.27 millimeters (mm). This setting may vary according to the thickness of the processed material and other factors.

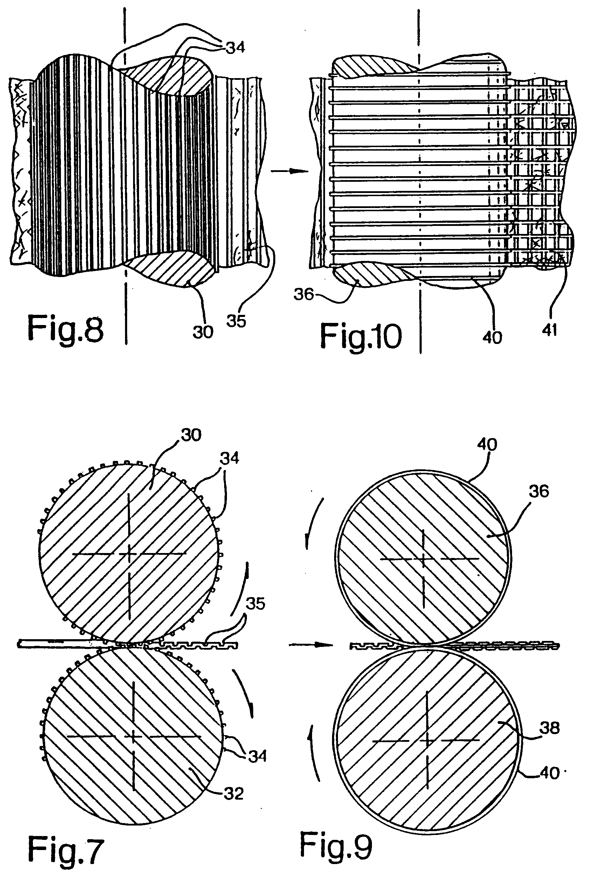

The second step of the perf-embossing operation consists of embossing the perforated, non-defibrated cellulosic board in the cross-direction by passing the board between a pair of rolls 30,32 with intermeshing longitudinally extending flutes 34. Figures 7 and 8 best illustrate the cross-direction embossing rolls 30 and 32 and the structure imparted to the non-defibrated cellulosic board. The flutes 34 imprint lines 35 on each surface of the material by locally compacting the material under the effect of mechanical compression.

In a preferred embodiment, the interference, i.e. the overlap between the flutes 34 of the rolls 30,32 is set at approximately 0.89 mm. This setting may vary according to the specific operating conditions.

The last step of the perf-embossing operation consists in embossing the resulting material in the machine direction by passing the web between parallel rolls 36,38 having circumferentially extending and intermeshing flutes 40, as best shown in Figures 9 and 10. This means a perpendicular impact to the second step operation, creating longitudinal lines 41.

In a preferred embodiment, the interference between the machine direction embossing rolls 36,38 is set at 0.76 mm. This setting may vary according to the specific operating conditions.

The perf-embossing treatment contributes to provide desirable mechanical properties to the non-defibrated cellulosic board, such as an increased flexibility enhancing the comfort potential of the product. The slits made at the perforation stage contribute to open-up the fibrous structure at precise locations, thus locally disrupting fiber bonds to render the material more pliable. The lines 35 and 41 constitute miniature hinges, extending throughout the entire surface of the non-defibrated cellulosic board to render the material more compliant in a transverse and in a longitudinal direction.

An alternative to the perf-embossing technique is the microcorrugating operation which is similar to the perf-embossing except that no perforations are performed. The fluid-absorbent structure is solely subjected to an embossing operation to create closely spaced hinge lines. The microcorrugating operation is described in U.S. patents granted to Personal Product Company, numbers 4,596,567 and 4,559,050, issued on June 24, 1986 and December 17, 1988, respectively.

The invention will now be illustrated by examples. The examples are not intended to be limiting of the scope of the present invention but read in conjunction with a detailed and general description above provide a further understanding of the present invention.

The physical and the fluid-absorbency characteristics of the sample materials obtained by the following examples are reported in the section entitled "EXPERIMENTAL DATA". The various test procedures to which the sample materials have been subjected for the purpose of characterization are described in the section entitled "TEST PROCEDURES".

### Example 1

Kraft wood pulp treated with debonding agent which is commercially available from the Weyerhaeuser Company under the name NBFA is dispersed in water to form a slurry.

Cross-linked cellulosic fibers are prepared in accordance with the teaching of the U.S. patent 4,853,086, mentioned earlier. The cross-linked cellulosic fibers are added to the slurry in a proportion of 25% by weight of de-moisturized cellulose in the slurry. The slurry is de-watered on a foraminous plate and the resulting web is dried, calendered at 4027 Newtons per centimeter (N/cm) and tenderized by the perf-embossing technique described earlier.

### Example 2

The same procedure set forth in example 1 is followed except that the proportion of cross-linked cellulosic fibers in the aqueous slurry is increased to 50% by weight of de-moisturized cellulose in the slurry.

### Example 3

The same procedure set forth in example 1 is followed except that the proportion of cross-linked cellulosic fibers in the aqueous slurry is increased to 75% by weight of de-moisturized cellulose in the slurry.

### Example 4

The same procedure set forth in example 1 is followed except that the perf-embossing operation of the non-defibrated cellulosic board is omitted.

### Example 5

The same procedure set forth in example 4 is followed except that the proportion of cross-linked cellulosic fibers in the aqueous slurry is increased to 50% by weight of de-moisturized cellulose in the slurry.

### Example 6

The same procedure set forth in example 4 is followed except that the proportion of cross-linked cellulosic fibers in the aqueous slurry is increased to 75% by weight of de-moisturized cellulose in the slurry.

### Example 7

The same procedure as set forth in example 1 is followed except that the calendering and the perf-embossing operations are omitted.

### Example 8

The same procedure set forth in example 7 is followed except that the proportion of cross-linked cellulosic fibers in the aqueous slurry is increased to 50% by weight of de-moisturized cellulose in the slurry.

### Example 9

The same procedure set forth in example 7 is followed except that the proportion of cross-linked cellulosic fibers in the aqueous slurry is increased to 75% by weight of de-moisturized cellulose in the slurry.

In order to provide a basis for comparison of the fluid absorption properties of the sample materials under examples 1 to 9, three control samples are provided which have a composition in accordance with the prior art.

### CONTROL 1

A slurry of 100% NBFA brand Kraft wood pulp is prepared. The slurry is sheeted, dried, calendered at 4027 N/cm and perf-embossed.

### CONTROL 2

The same procedure as set forth in Control 1 is followed except that the perf-embossing is omitted.

### CONTROL 3

The same procedure as set forth in Control 1 is followed except that the calendering and the perf-embossing are omited.

The materials of examples 1 to 9 and of the controls 1 to 3 are tested to assess the following characteristics:
a) absorbent capacity (expressed in cubic centimeters per gram [cc/g]) by the gravimetric absorbency test system (GATS) at three different pressures (identified by GATS1, GATS2, and GATS3 in the following table) applied on the sample;
b) drying power (expressed in cc/g);
c) 45° impact capacity (expressed in percentage);
d) tensile strength (expressed in Newtons per centimeter [N/cm]);
e) density (expressed in grams per cubic centimeter [g/cc]).

| **EXPERIMENTAL DATA** | | | | | | | |
|---|---|---|---|---|---|---|---|
| **Examples** | **GATS 1 (cc/g)** | **GATS 2 (cc/g)** | **GATS 3 (cc/g)** | **DRYING POWER (cc/g)** | **45% IMPACT CAPACITY (%)** | **TENSILE STRENGTH (N/cm)** | **DENSITY (g/cc)** |
| 1 | 7.6 | 4.9 | 6.8 | 4 | 77 | 1.82 | 0.164 |
| 2 | 9.2 | 5.1 | 7.7 | 5.9 | 93 | 0.91 | 0.137 |
| 3 | 10.1 | 5.5 | 8.5 | 7.6 | 92 | 0.18 | 0.129 |
| 4 | 6.6 | 4.5 | 6.2 | 6.7 | 69 | 8.77 | 0.331 |
| 5 | 7.5 | 4.8 | 6.9 | 7.8 | 73 | 4.67 | 0.272 |
| 6 | 9.1 | 6 | 8.3 | 7.8 | 86 | 1.45 | 0.223 |
| 7 | 8.3 | 6.3 | 7.9 | 8.7 | 86 | 7.63 | 0.107 |
| 8 | 9.4 | 6.9 | 8.9 | 9.4 | 84 | 4.36 | 0.083 |
| 9 | 11.9 | 7.3 | 10.6 | 9.7 | 97 | 1.09 | 0.075 |
| Control 1 | 6.9 | 4.8 | 6.1 | 3.3 | 78 | 0.56 | 0.166 |
| Control 2 | 5.6 | 4.0 | 5.0 | 7.7 | 60 | 10.33 | 0.251 |
| Control 3 | 6.5 | 4.9 | 6.3 | 7.9 | 66 | 10.92 | 0.142 |

### DESCRIPTION OF TEST PROCEDURES

### GATS 1

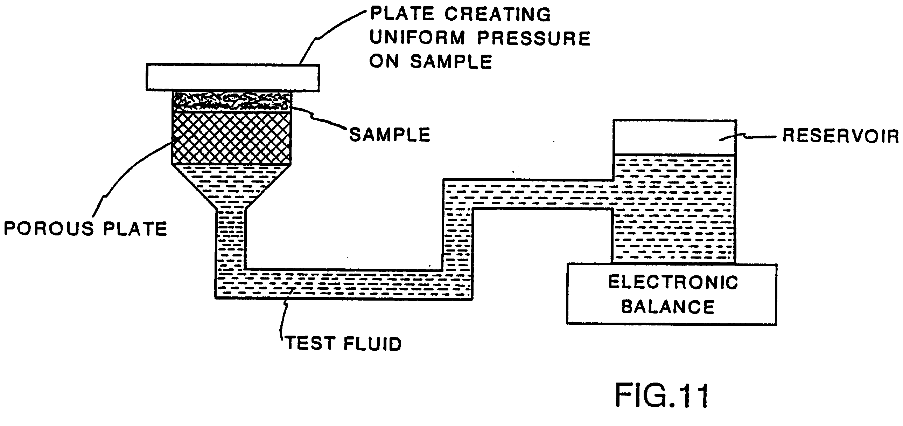

- **Purpose:**: To determine the ultimate fluid absorption capacity of a sheeted material under light pressure.
- **Test Procedure:**: With reference to Figure 11, the sample is deposited on a porous plate in fluid communication with a reservoir whose weight is continuously measured by an electronic balance. A uniform pressure of 0.069 kiloPascals (kPa) is applied on the sample. The amount (volume) of fluid absorbed by the sample from the reservoir via the porous plate after 15 minutes is recorded and divided by the weight of the sample to normalize the capacity per unit of mass of the sample. An apparatus for automatically performing this test is commercially available from M/K Systems Inc.
- **Test Fluid:**: 1% NaCl solution.

### GATS 2

- **Purpose:**: To determine the ultimate absorbent capacity of a sheeted material under a significant pressure.
- **Test Procedure:**: The same set-up as the test GATS1 is used. Upon completion of the test GATS1, when the sample is fully loaded with fluid, the pressure on the sample is increased to 3.448 kPa. The amount of fluid remaining in the sample is calculated by determining the amount of reverse flow toward the reservoir, and normalized per unit of mass of the sample.
- **Test Fluid:**: 1% NaCl solution.

### GATS 3

- **Purpose:**: To determine the resiliency of a sheeted material which is loaded with fluid.
- **Test Procedure:**: The same set-up as the test GATS2 is used. Upon completion of the test GATS2, the amount of pressure on the sample is reduced to 0.069 kPa. The amount of fluid re-absorbed by the sample is measured and normalized per unit of mass of the sample. The resulting value is an indication of the normalized internal volume re-acquired by the sample as a result of the pressure decrease which provides an indication of the resiliency of the sample in a wet state.

### DRYING POWER

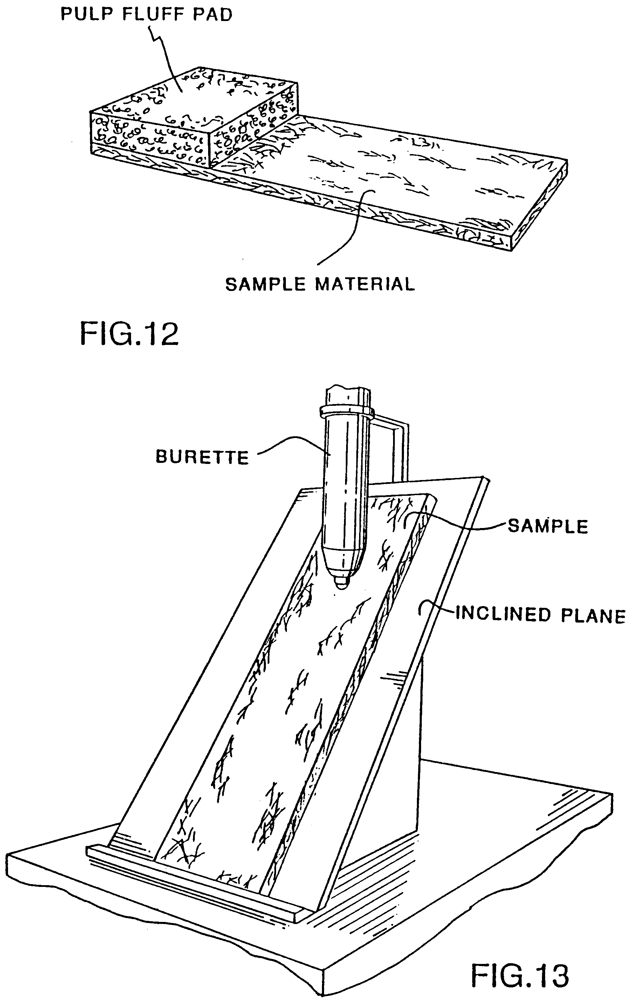

- **Purpose:**: To determine the dewatering ability or the desorbing power of an absorbent material by capillary migration of a test fluid away from an impact zone.
- **Test procedure:**: With reference to Fig. 12, a pad of pulp fluff of 5 cm x 5 cm having a basis weight of 120 g/m² is deposited on the extremity of a strip of the sample material whose dimensions are of 5 cm x 18 cm. A volume of test liquid equivalent to 3 cc/g based on the weight of the strip of sample material is discharged on the pad of pulp fluff. The amount of fluid retained in the pad of pulp fluff after 30 minutes is determined by weight difference of the pulp fluff pad at the beginning and at the end of the test and normalized per unit of mass of the pulp fluff pad.
- **Test Fluid:**: 1% NaCl solution.

### 45° IMPACT CAPACITY

- **Purpose:**: To determine the fluid retention capacity of an absorbent material by measuring its ability to accept and retain a finite discharge of fluid at an inclined plane.
- **Test procedure:**: With reference to Figure 13, the impact capacity of a 10 cm x 18 cm sample is measured by weighing the amount of fluid that is retained in the sample placed on a 45° inclined plane, on which an amount of test fluid corresponding to ten times the sample weight has been released from an overhanging burette. The burette barely touches the sample at a point approximately 6.5 cm away from its upper extremity. The amount of fluid retained in the sample is expressed in terms of percentage of the total amount of fluid delivered.
- **Test fluid:**: 1% NaCl solution

### TENSILE STRENGTH

- **Purpose:**: To determine the structural strength of the absorbent material by measuring the force required to rupture the sample.
- **Test procedure:**: The tensile strength is measured by recording the force required to rupture a 2.5 cm wide sample placed between two jaws 7.6 cm apart and moving apart at a continuous rate.

### DENSITY

- **Purpose****:**: To determine the density of an absorbent material under a predetermined pressure.
- **Test procedure:**: The density is obtained by measuring the weight of the sample and dividing it by its volume (thickness X area of sample).

### THICKNESS

- **Purpose****:**: To measure the thickness of an absorbent material for determining its density.
- **Test procedure:**: 1) Non-defibrated and non-tenderized board: The thickness of the board material is measured with a TMI thickness gauge at 48.26 kPa with a 1.59 cm diameter foot (TAPPI standard T411 0S-76);
2) Non-defibrated tenderized board: The thickness of the sample is measured at 0.345 kPa with a compressometer using a 5.1 cm diameter foot (ASTM D-1777).

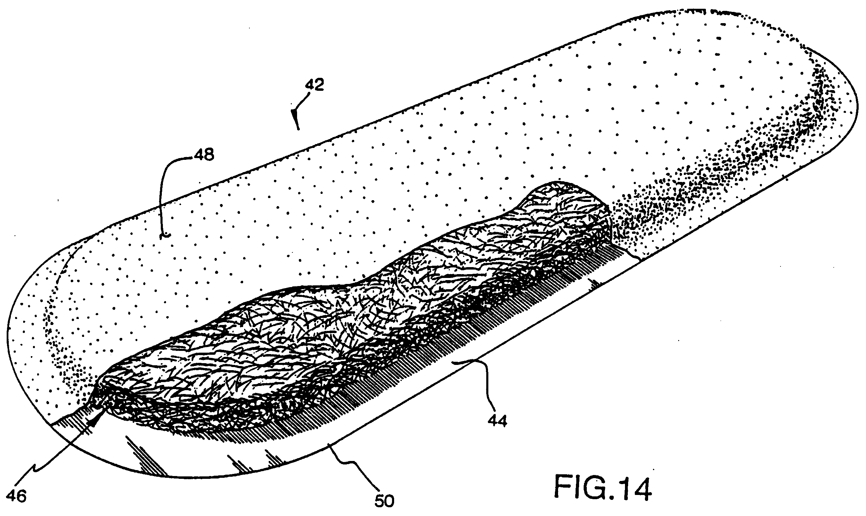

Figure 14 illustrates a sanitary napkin incorporating the non-defibrated cellulosic board according to the invention. The sanitary napkin, designated comprehensively by the reference numeral 42, comprises an envelope 44 defining an internal space receiving an absorbent component 46. The envelope 44 includes a fluid-permeable cover layer 48 made of non-woven fabric or any other suitable porous web, and a liquid-impervious backing layer 50 made of polyethylene film, for example. The cover and backing layers 48 and 50 are heat-sealed to one another along their marginal portions.

The absorbent component 46 may have a single or a double layer configuration. In the former case, depicted in Figure 14, the absorbent component 46 is an insert constituted by the non-defibrated cellulosic board according to the invention. In the latter case (not shown in the drawings), the absorbent component has a top layer, referred to as a transfer layer and a bottom layer, designated as a reservoir layer. The layers are in a superposed relationship. The non-defibrated cellulosic board is particularly suitable for use as a transfer layer due to its high fluid-acceptance rate, coupled in an intimate fluid-communicative relationship with a reservoir layer made of any suitable highly absorbent and retentive material. In a variant, the non-defibrated cellulosic board may be adapted for reservoir layer duty or use by increasing its density, as previously described.

To attach the sanitary napkin 42 to the wearer's underpants, the fluid-impervious backing layer 50 is provided with adhesive zones covered with a peelable backing (not shown in the drawings).

Sanitary napkins constructed in accordance with the present invention are found to possess a very high fluid-absorption capacity and a comparatively high fluid-acceptance rate which reduces the risk of failure when a large quantity of fluid is suddenly released on the sanitary napkin. In addition, the sanitary napkin is thin, soft and flexible so as to be comfortable to the user and to conform well to the surface of the body to which it is applied to achieve good gasketing effects.

Applications of the product and methods of the present invention for sanitary and other health-care uses can be accomplished by any sanitary protection, incontinence, medical and absorbent methods and techniques that are presently or prospectively known to those skilled in the art. Thus, it is intended that the present application covers the modifications and variations of this invention provided that they come within the scope of the appended claims and their equivalents.

## Claims

1. A fluid-absorbent sheet consisting of a non-defibrated, cellulosic board characterized by said board containing effective amounts of debonding agent and cross-linked cellulosic fibers.

2. A fluid-absorbent sheet as defined in claim 1, wherein said non-defibrated cellulosic board is mechanically tenderized to further enhance its flexibility.

3. A fluid-absorbent sheet as defined in claim 2, wherein said non-defibrated cellulosic board is mechanically tenderized by a process selected from the group consisting of perf-embossing and microcorrugating.

4. A fluid-absorbent sheet as defined in claim 1, wherein said non-defibrated cellulosic board contains cross-linked cellulosic fibers in the range from about 20 to about 80 percent based on the weight of de-moisturized cellulose in said board.

5. A fluid-absorbent sheet as defined in claim 1, wherein said cross-linked cellulosic fibers comprise cellulosic fibers treated with a solution of a glycol and dialdehyde.

6. A fluid-absorbent sheet as defined in claim 1, wherein said debonding agent is present in said non-defibrated cellulosic board in the range from about 0.05 to about 5 percent based on the weight of the de-moisturized board.

7. A fluid-absorbent sheet as defined in claim 1, wherein said debonding agent is hydrophilic.

8. A fluid-absorbent sheet as defined in claim 1, wherein said non-defibrated cellulosic board is calendered.

9. A fluid-absorbent sheet as defined in claim 1, wherein said non-defibrated cellulosic board includes a material selected from the group consisting of sulfate, sulfite, debonded, bleached or unbleached wood pulp, thermo-mechanical pulp, chemical thermal mechanical pulp, ground wood, cotton linters and mixtures thereof.

10. A method for manufacturing a fluid-absorbent, resilient and flexible sheet from a non-defibrated cellulosic board, comprising the step of incorporating in said non-defibrated cellulosic board effective amounts of cross-linked cellulosic fibers and debonding agent.

## Patentansprüche

1. Flüssigkeitsabsorbierende Lage, bestehend aus einer nicht-zerfaserten Cellulosepappe, dadurch gekennzeichnet, daß die Pappe wirksame Mengen an bindungslösendem Mittel und vernetzten Cellulosefasern enthält.

2. Flüssigkeitsabsorbierende Lage nach Anspruch 1, wobei die nicht-zerfaserte Cellulosepappe mechanisch geschwächt ist, um ihre Flexibilität weiter zu erhöhen.

3. Flüssigkeitsabsorbierende Lage nach Anspruch 2, wobei die nicht-zerfaserte Cellulosepappe mechanisch nach einem Verfahren geschwächt ist, das aus der Gruppe Perforationsprägung und Mikroriffelung ausgewählt ist.

4. Flüssigkeitsabsorbierende Lage nach Anspruch 1, wobei die nicht-zerfaserte Cellulosepappe vernetzte Cellulosefasern im Bereich von etwa 20 bis etwa 80%, bezogen auf das Gewicht der entfeuchteten Cellulose in der Pappe, enthält.

5. Flüssigkeitsabsorbierende Lage nach Anspruch 1, wobei die vernetzten Cellulosefasern mit einer Lösung eines Glykols und eines Dialdehyds behandelte Cellulosefasern umfassen.

6. Flüssigkeitsabsorbierende Lage nach Anspruch 1, wobei das bindungslösende Mittel in der nicht-zerfaserten Cellulosepappe in einer Menge im Bereich von etwa 0,05 bis etwa 5%, bezogen auf das Gewicht der entfeuchteten Pappe, enthalten ist.

7. Flüssigkeitsabsorbierende Lage nach Anspruch 1, wobei das bindungslösende Mittel hydrophil ist.

8. Flüssigkeitsabsorbierende Lage nach Anspruch 1, wobei die nicht-zerfaserte Cellulosepappe kalandriert ist.

9. Flüssigkeitsabsorbierende Lage nach Anspruch 1, wobei die nicht-zerfaserte Cellulosepappe ein Material umfaßt, das aus der Gruppe Sulfat-, Sulfit-, entklebter ("debonded"), gebleichter oder ungebleichter Holzzellstoff, thermomechanischer Zellstoff, chemisch-thermomechanischer Zellstoff, Holzschliff, Baumwoll-Linter und Gemische davon ausgewählt ist.

10. Verfahren zur Herstellung einer flüssigkeitsabsorbierenden, elastischen und flexiblen Lage aus einer nicht-zerfaserten Cellulosepappe, umfassend die Stufe, bei der der nicht-zerfaserten Cellulosepappe wirksame Mengen an vernetzten Cellulosefasern und bindungslösendem Mittel einverleibt werden.

## Revendications

1. Une feuille absorbant du fluide composée d'une plaque cellulosique non défibrée, caractérisée en ce que ladite plaque contient des quantités efficaces d'agent de rupture de liaisons et de fibres cellulosiques réticulées.

2. Une feuille absorbant du fluide telle que définie dans la Revendication 1, dans laquelle ladite plaque cellulosique non défibrée est assouplie de façon mécanique pour améliorer plus encore sa flexibilité.

3. Une feuille absorbant du fluide telle que définie dans la Revendication 2, dans laquelle ladite plaque cellulosique non défibrée est assouplie de façon mécanique par un procédé sélectionné parmi le groupe composé d'un gaufrage par perforation et d'un micronervurage.

4. Une feuille absorbant du fluide telle que définie dans la Revendication 1, dans laquelle ladite plaque cellulosique non défibrée contient des fibres cellulosiques réticulées dans la gamme comprise entre environ 20 et environ 80 pour-cent en masse sur la base de la masse de cellulose déshumidifiée comprise dans ladite plaque.

5. Une feuille absorbant du fluide telle que définie dans la Revendication 1, dans laquelle lesdites fibres cellulosiques réticulées comprennent des fibres cellulosiques traitées avec une solution d'un glycol et de dialdéhyde.

6. Une feuille absorbant du fluide telle que définie dans la Revendication 1, dans laquelle ledit agent de rupture de liaisons est présent dans ladite plaque cellulosique non défibrée dans la gamme comprise entre environ 0,05 et environ 5 pour-cent en masse sur la base de la masse de la plaque déshumidifiée.

7. Une feuille absorbant du fluide telle que définie dans la Revendication 1, dans laquelle ledit agent de rupture de liaisons est hydrophile.

8. Une feuille absorbant du fluide telle que définie dans la Revendication 1, dans laquelle ladite plaque cellulosique non défibrée est calandrée.

9. Une feuille absorbant du fluide telle que définie dans la Revendication 1, dans laquelle ladite plaque cellulosique non défibrée comprend un matériau sélectionné parmi le groupe composé de sulfate, de sulfite, de pâte de cellulose dont les liaisons ont été rompues, blanchie ou non blanchie, de pâte thermomécanique, de pâte chimique thermomécanique, de coton, de linters de coton et de mélanges de ceux-ci.

10. Une méthode de fabrication d'une feuille absorbant du fluide résiliente et souple à partir d'une plaque cellulosique non défibrée, comprenant l'étape consistant à incorporer dans ladite plaque cellulosique non défibrée des quantités efficaces de fibres cellulosiques réticulées et d'agent de rupture de liaisons.
